# FASCICULE DE BREVET EUROPEEN

(11) **EP 0 946 152 B1**
(45) Date de publication et mention de la délivrance du brevet: **14.11.2001**
(21) Numéro de dépôt: 97952083.0
(22) Date de dépôt: 17.12.1997
(51) Int. Cl.: A61K 9/48, A61K 9/50

(54) **CAPSULE BIODEGRADABLE A BASE D'UNE PROLAMINE**
BIODEGRADIERBARE PROLAMINKAPSEL
BIODEGRADABLE CAPSULE WITH A PROLAMIN BASE

(30) Priorité: 18.12.1996 FR 9615547
(43) Date de publication de la demande: 06.10.1999
(73) Titulaire: Isocell, 92200 Neuilly sur Seine (FR)
(72) Inventeur: DUGAS, Bernard, F-95150 Taverny (FR); CALENDA, Alphonse, F-92100 Boulogne (FR); LATI, Elian, F-92170 Vanves (FR)
(74) Mandataire: Mouget-Goniot, Claire
(86) Numéro de dépôt international: FR9702325
(87) Numéro de publication internationale: WO9826766

(56) Documents cités:
- EP-A- 0 447 297
- EP-A- 0 585 688
- WO-A-93/12771
- FR-A- 2 710 649
- US-A- 4 137 300
- Lehrbuch der pharmazeutischen Technologie, Rudolf Voigt, 5ème édition, Verlag Chemie, 1984, ISBN: 3-527-26112-5, pages 227-233

## Description

La présente invention est relative à une nouvelle formulation galénique de type gélule ou capsule molle comprenant un substitut de la gélatine.

Jusqu'à présent, on utilise pour fabriquer des gélules ou des capsules molles, de la gélatine d'origine bovine, qui présente des risques de transmission de virus ou d'éléments infectieux.

Par ailleurs, la Demande de Brevet FR 2 710 649 décrit un film polymérique à base de prolamines qui peut être utilisé dans le domaine pharmaceutique comme support ou vecteur de substances actives.

Les Inventeurs se sont donné pour but de pourvoir à un substitut de la gélatine qui réponde mieux aux besoins de la pratique, notamment en ce qu'il présente au moins les mêmes caractéristiques de biodisponibilité que celles de la gélatine.

La présente invention a pour objet des capsules biodégradables à effet retard et à libération progressive, caractérisées en ce qu'elles sont constituées :
- d'un noyau choisi dans le groupe consistant en :
   - une poudre micronisée d'au moins un principe actif enrobé dans au moins une prolamine d'origine végétale,
   - une poudre micronisée d'au moins un principe actif enrobé dans au moins une prolamine d'origine végétale, en suspension dans une solution huileuse, et
- d'une enveloppe constituée par un film polymérique comprenant une prolamine d'origine végétale, dont la composition est la suivante (en p/p) : 60 à 75 % de prolamine, 20 à 35 % de plastifiant et 2 à 5 % d'eau.

De telles capsules biodégradables, d'origine végétale, constituent un substitut avantageux aux capsules de gélatine ; en particulier, elles ne présentent pas les inconvénients et les risques de transmission de virus ou d'éléments infectieux des supports d'origine animale, tout en présentant une consistance proche des capsules de l'Art antérieur à base de gélatine ; elles présentent, en outre, d'autres avantages et notamment celui de contrôler la biodisponibilité des différents principes actifs qui peuvent lui être associés (substances chimiques ou naturelles, protéines, acides nucléiques etc.).

Selon un mode de réalisation avantageux desdites capsules, ladite prolamine est une prolamine native (c'est-à-dire non dénaturée), issue soit de la farine, soit du gluten frais d'une céréale, telle que le blé, le maïs, l'orge, l'avoine ou le seigle, de préférence le blé (gliadine) ou le maïs (zéine).

Selon un autre mode de réalisation avantageux desdites capsules, ledit film polymérique de prolamine formant l'enveloppe desdites capsules est susceptible d'être obtenu après évaporation du solvant, à partir d'une solution liquide de départ qui comprend (en poids/poids) entre 40 à 80 % d'un solvant hydro-alcoolique dont le titre en alcool est compris entre 40 et 80 %, 20 à 50 % d'au moins une prolamine et au moins un plastifiant, le rapport plastifiant/solution hydro-alcoolique de prolamine étant compris entre 0,1/1 et 0,5/1.

Ladite solution comprend de préférence (en poids/poids) 40 à 60 % de solution hydro-alcoolique, 25 à 30 % de prolamine et 10 à 30 % de plastifiant.

De préférence :
- le solvant est sélectionné dans le groupe constitué par les monools, les diols et l'eau tels que définis dans cette Demande française n° 2 710 649 et
- le plastifiant est sélectionné dans le groupe constitué par les polyols et les esters tels que phtalates, adipates, sébaçates, phosphates, citrates, tartrates et malates ; de préférence ledit plastifiant est sélectionné dans le groupe constitué par le glycérol, le sorbitol, le xylitol, le néosorb® 70/70 (mélange contenant au moins 70 % de sorbitol et de l'eau, produit Roquette), le PEG400 et le PEG1000 ; de préférence, le plastifiant est un mélange de sorbitol et de glycérol (correspondant à 10-18 % de la solution de prolamine de départ), dans un rapport compris entre 1/1 et 2,5/1, de préférence compris entre 2/1 et 2,5/1.

Les films polymériques secs de prolamine, et notamment de gliadine, sont obtenus par évaporation du solvant, dans les conditions décrites dans la Demande de Brevet français n° 2 710 649.

Conformément à l'invention, l'évaporation du solvant permet d'obtenir un gel de prolamine, dont la formule exprimée en poids/poids est la suivante : 35 à 45 % de prolamine, 10 à 26 % de plastifiant, et une solution hydro-alcoolique pour compléter à 100 %.

A partir de ce gel, on obtient un film.

Conformément à l'invention, pour l'obtention de gélules ou capsules dures, le noyau est constitué d'une poudre micronisée d'au moins un principe actif enrobé dans au moins une prolamine d'origine végétale.

Ledit mélange est obtenu comme suit : dissolution du (des) principes actif(s) dans la solution hydro-alcoolique, addition à une température comprise entre 30 et 45°C, de la prolamine sous forte agitation pour former un gel, évaporation du solvant sous vide et micronisation du résidu solide ; dans ce type de noyau, le (les) principe(s) actif(s) est (sont) enrobé(s) dans de la prolamine.

Également conformément à l'invention, pour l'obtention de capsules molles, le noyau est constitué d'une poudre micronisée d'au moins un principe actif enrobé dans au moins une prolamine d'origine végétale, en suspension dans une solution huileuse, l'huile étant une de celles habituellement utilisée dans les formulations huileuses (huile d'olive, de tournesol, de poisson). Dans ce cas, ladite poudre micronisée est obtenue comme décrit précédemment pour les capsules dures.

De manière surprenante, les capsules selon l'invention permettent d'obtenir à la fois une protection contre les effets délétères du pH gastrique et une libération par effet retard, ainsi qu'une libération progressive du principe actif au niveau du système intestinal ; elles permettent, en outre, d'optimiser la biodisponibilité des principes actifs associés et de résoudre le problème de l'administration par voie orale de molécules qui seraient détruites au niveau du système gastro-intestinal, ce qui est notamment le cas pour les protéines, les peptides et les acides nucléiques, etc.

Elles constituent ainsi à la fois un substitut des gélules et capsules molles déjà existantes à base de gélatine bovine mais également un nouveau support et/ou vecteur de principe actif par voie orale.

Les capsules selon l'invention, également dénommées FRACTOCAPS® , permettent effectivement : 1) une substitution avantageuse de la gélatine bovine par une protéine végétale et 2) une amélioration de la biodisponibilité et de la libération de substances actives.

Outre les dispositions qui précèdent, l'invention comprend encore d'autres dispositions, qui ressortiront de la description qui va suivre, qui se réfere à des exemples de mise en oeuvre du procédé objet de la présente invention ainsi qu'aux dessins annexés, dans lesquels :
- la figure 1 illustre la biodisponibilité du saquinavir à partir de capsules de gélatine contenant du saquinavir,
- la figure 2 illustre la biodisponibilité du saquinavir compris dans des capsules molles de gliadine, dans lesquelles l'enveloppe est constituée d'un film polymérique de gliadine, l'enveloppe de la capsule de gliadine-2 étant deux fois plus épaisse que celle de la capsule de gliadine-1 ; dans ces capsules molles, le noyau interne est constitué de saquinavir libre sous forme de poudre micronisée, en suspension dans une solution huileuse,
- la figure 3 illustre la biodisponibilité du saquinavir à partir d'un gel polymérique de gliadine comprenant du saquinavir (pas d'encapsulation), la pâte molle de gliadine-2 étant deux fois plus concentrée en gliadine que la pâte molle de gliadine-1,
- la figure 4 illustre une comparaison de la libération du saquinavir à partir de capsules de gélatine ou d'un gel de gliadine contenant le principe actif et transformé en pâte molle et des capsules suivantes :

- capsules molles de gliadine dans lesquelles le noyau est sous forme d'une suspension huileuse de principe actif libre, et
- capsules dures (gélules) comportant une enveloppe constituée d'un film polymérique sec de gliadine et d'un noyau comprenant le principe actif enrobé dans de la gliadine, sous forme d'une poudre micronisée.

Il doit être bien entendu, toutefois, que ces exemples sont donnés uniquement à titre d'illustration de l'objet de l'invention, dont ils ne constituent en aucune manière une limitation.

### EXEMPLE 1 : Gélules de saquinavir selon l'invention.

### 1. préparation de l'enveloppe de la gélule :

on prépare un gel de gliadine à partir d'une solution qui comprend :
. 57,8 % (p/p) d'un mélange eau/éthanol (1/1) (solvant),
. 27,2 % de gliadine (p/p),
. 15 % de plastifiants (p/p) comprenant du sorbitol et du glycérol dans un rapport 70/30 (v/v).

Le gel est séché ; on obtient ainsi un film sec de polymère de gliadine ; les films seront associés, pour former la capsule finale.

De manière générale, le film polymérique de gliadine (gel ou film sec) peut être préparé selon les différentes modalités décrites dans la Demande française 2 710 649.

### 2. préparation du noyau : mélange sous forme de poudre micronisée de saquinavir enrobé dans de la gliadine :

145 mg de saquinavir, qui est un inhibiteur de protéase et qui est utilisé dans le traitement de l'infection par le virus VIH-1, sont dissous dans 1 à 1,25 g de solvant hydro-alcoolique (mélange eau/éthanol ; 0,5 à 0,6 g de poudre de gliadine est ajoutée sous forte agitation, à 40°C, pour former un gel (environ 2 g).

Le gel obtenu, une fois homogène, est coulé sur un support en polypropylène et séché (stabilisation à 60°C et 2 % d'humidité relative, pendant 48 heures). Le produit sec obtenu est ensuite micronisé et introduit dans les capsules, telles que préparées en 1 et hermétiquement scellées.

### EXEMPLE 2 : Capsules molles de saquinavir selon l'invention.

### 1. préparation de l'enveloppe :

on procède comme à l'exemple 1.

### 2. préparation du noyau :

La poudre micronisée telle qu'obtenue à l'exemple 1 (2.) est mise en suspension dans une solution huileuse (solution huileuse à 5-15 %).

La suspension huileuse obtenue est coulée dans l'enveloppe de gliadine, puis la capsule obtenue est scellée.

### EXEMPLE 3 : Étude comparée de biodisponibilité

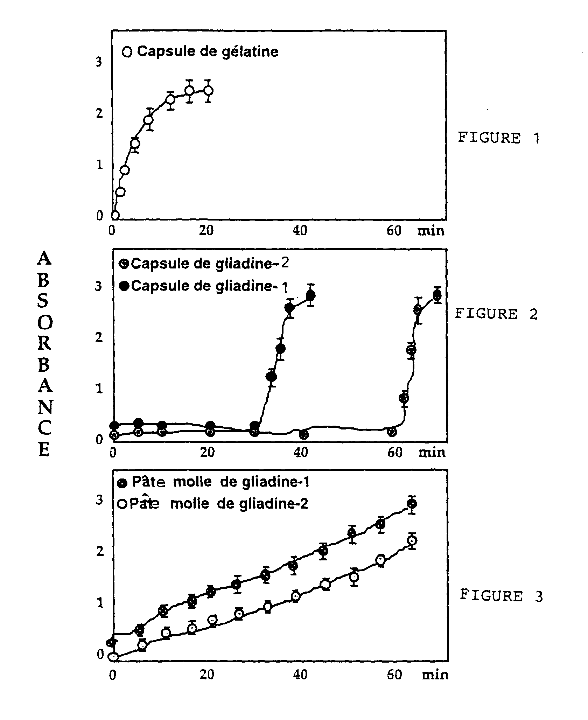

Les résultats illustrés aux figures 1, 2, 3 et 4 sont obtenus en plaçant les différentes capsules dans un milieu mimant le pH gastrique et en comparant le processus de libération du saquinavir dans les différentes formes galéniques. La libération du produit est évaluée par spectrophotométrie à 238 nm.

De manière plus précise, la simulation de dissolution et de biodisponibilité du saquinavir est réalisée dans un bain thermostaté à 37°C et le suivi de cette dissolution est réalisé de manière continue dans un milieu mimant le pH gastrique (acide chlorhydrique 0,1 N, pH = 1,2). L'absorbance à 238 nm est réalisée toutes les 2 minutes jusqu'à 60 min, puis toutes les 5 minutes.

Les résultats présentés aux figures 1, 2, 3 et 4 démontrent que lorsque le saquinavir est encapsulé dans une enveloppe à base de gliadine (figure 2), le principe actif étant libre dans le noyau, le produit est libéré après 30 à 60 min à pH gastrique (le temps le plus long correspondant à l'enveloppe de gliadine la plus épaisse), alors que dans la formulation avec la gélatine, cette libération est beaucoup plus rapide puisque le produit est libéré dans les 2 à 3 min (figure 1) qui suivent la mise en contact avec le milieu à pH gastrique. L'encapsulation du principe actif dans une enveloppe de gliadine permet donc une libération retardée.

Lorsque le saquinavir est incorporé dans une pâte molle de gliadine (pas d'encapsulation), sa libération en milieu pH gastrique est progressive puisqu'elle débute rapidement (dans les 5 min) et se prolonge au delà d'une heure indiquant une libération progressive du principe actif (figure 3).

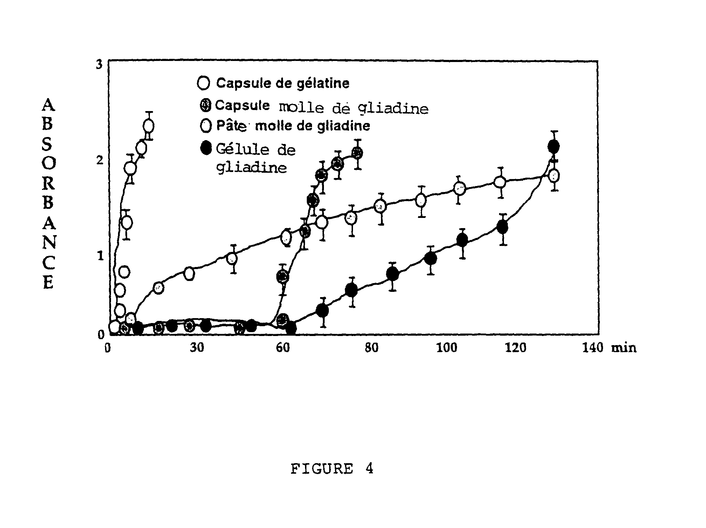

Lorsque le saquinavir est enrobé dans de la gliadine et encapsulé dans une enveloppe constituée d'un film polymérique sec de gliadine (Figure 4), ce qui correspond à une gélule ou capsule dure selon l'invention, la libération du principe actif se fait en deux étapes : après dissolution de l'enveloppe de polymère de gliadine (environ 60 minutes), on observe une libération progressive du principe actif (de 60 minutes à 120 minutes et plus) par dissolution progressive du gel de gliadine. Ainsi, les deux effets de libération, retardée et progressive, sont cumulés.

Dans la présente invention, le saquinavir est utilisé à titre d'exemple et ces résultats ne sont en aucun cas restrictifs. En effet, la formulation selon l'invention peut être adaptée à tout type de substance thérapeutique, de principe actif, de protéine, de peptide, de dérivés nucléotidiques, de vaccins et de produits nutritifs.

## Revendications

1. Capsules biodégradables à effet retard et à libération progressive, **caractérisées en ce qu'**elles sont constituées :
- d'un noyau choisi dans le groupe consistant en :
- une poudre micronisée d'au moins un principe actif enrobé dans au moins une prolamine d'origine végétale,
- une poudre micronisée d'au moins un principe actif enrobé dans au moins une prolamine d'origine végétale, en suspension dans une solution huileuse, et
- d'une enveloppe constituée par un film polymérique comprenant une prolamine d'origine végétale, dont la composition est la suivante (en p/p) : 60 à 75 % de prolamine, 20 à 35 % de plastifiant et 2 à 5 % d'eau.

2. Capsules selon la revendication 1, **caractérisées en ce que** ladite prolamine est une prolamine native, issue soit de la farine, soit du gluten frais d'une céréale, telle que le blé, le maïs, l'orge, l'avoine ou le seigle, de préférence le blé ou le maïs, de préférence de la gliadine.

3. Capsules biodégradables selon la revendication 1 ou la revendication 2, **caractérisées en ce que** ledit film polymérique comprenant une prolamine est susceptible d'être obtenu après évaporation du solvant, à partir d'une solution liquide de départ qui comprend (en poids/poids) entre 40 à 80 % d'un solvant hydro-alcoolique dont le titre en alcool est compris entre 40 et 80 %, 20 à 50 % d'au moins une prolamine et au moins un plastifiant, le rapport plastifiant/solution hydro-alcoolique de prolamine étant compris entre 0,1/1 et 0,5/1.

4. Capsules biodégradables selon la revendication 3, **caractérisées en ce que** ledit film polymérique comprenant une prolamine est de préférence obtenu à partir d'une solution de départ qui comprend (en poids/poids) 40 à 60 % de solution hydro-alcoolique, 25 à 30 % de prolamine et 10 à 30 % de plastifiant.

5. Capsules biodégradables selon l'une quelconque des revendications 1 à 4, **caractérisées en ce que** le plastifiant est un mélange de sorbitol et de glycérol dans un rapport compris entre 1/1 et 2,5/1, de préférence compris entre 2/1 et 2,5/1.

## Patentansprüche

1. Biologisch abbaubare Kapseln mit verzögerter Wirkung und progressiver Freisetzung,
**dadurch gekennzeichnet, dass** sie aus
- einem Kern, ausgewählt aus der Gruppe bestehend aus
- einem mikronisierten Pulver mindestens eines Wirkstoffs, der von mindestens einem Prolamin pflanzlicher Herkunft umhüllt ist,
- einem mikronisierten Pulver mindestens eines Wirkstoffs, der von mindestens einem Prolamin pflanzlicher Herkunft umhüllt ist, in Suspension in einer ölhaltigen Lösung, und
- einer Umhüllung, die aus einem Polymerfilm besteht, der ein Prolamin pflanzlicher Herkunft umfasst, dessen Zusammensetzung folgende (Gewicht/Gewicht) ist: 60 bis 75% Prolamin, 20 bis 35% Weichmacher und 2 bis 5% Wasser,
bestehen.

2. Kapseln nach Anspruch 1,
**dadurch gekennzeichnet, dass** das Prolamin ein natives Prolamin ist, das entweder von Mehl, frischem Gluten eines Getreides wie z. B. Weizen, Mais, Gerste, Hafer oder Roggen, vorzugsweise Weizen oder Mais, insbesondere von Gliadin stammt.

3. Biologisch abbaubare Kapseln nach Anspruch 1 oder Anspruch 2,
**dadurch gekennzeichnet, dass** der Polymerfilm, der ein Prolamin umfasst, fähig ist, ausgehend von einer flüssigen Ausgangslösung, die (G/G) von 40 bis 80% eines Wasser-Alkohol-Lösungsmittels, dessen Alkoholgehalt zwischen 40 und 80% liegt, von 20 bis 50% mindestens eines Prolamins und mindestens eines Weichmachers, wobei das Verhältnis Weichmacher/Wasser-Alkohol-Lösung von Prolamin zwischen 0,1/1 und 0,5/1 liegt, umfasst, nach Verdampfung des Lösungsmittels erhalten zu werden.

4. Biologisch abbaubare Kapseln nach Anspruch 3,
**dadurch gekennzeichnet, dass** der Polymerfilm, der ein Prolamin umfasst, vorzugsweise ausgehend von einer Ausgangslösung erhalten wird, welche (in Gewicht/Gewicht) von 40 bis 60% Wasser-Alkohol-Lösung, von 25 bis 30% Prolamin und 10 bis 30% Weichmacher umfasst.

5. Biologisch abbaubare Kapseln nach einem der Ansprüche 1 bis 4,
**dadurch gekennzeichnet, dass** der Weichmacher ein Gemisch aus Sorbit und Glyzerin in einem Verhältnis, das zwischen 1/1 und 2,5/1, vorzugsweise zwischen 2/1 und 2,5/1 liegt, ist.

## Claims

1. Delayed-effect and gradual-release biodegradable sealed capsules, **characterized in that** they consist:
- of a core selected from the group consisting of:
- at least one active principle wherein a micronized powder of the active principle is coated with at least one prolamine of plant material, and
- at least one active principle wherein a micronized powder of the active principle is coated with at least one prolamine of plant origin and then suspended in an oily solution; and
- a polymeric film envelope comprising one prolamine of plant origin, the composition of which is as follows, on a weight/weight basis, 60 to 75% prolamine, 20 to 35% plasticizer and 2 to 5% water.

2. Capsules according to claim 1, **characterized in that** the said prolamine is a native prolamine, obtained either from the flour or from the fresh gluten of a cereal, such as wheat, corn, barley, oat or rye, preferably wheat or corn, preferably gliadin.

3. Biodegradable capsules according to claim 1 or to claim 2, **characterized in that** the said polymeric film comprising one prolamine can be obtained after evaporation of the solvent, from a liquid starting solution which comprises (on a weight/weight basis) between 40 and 80% of an aqueous-alcoholic solvent whose alcohol titer is between 40 and 80%, 20 to 50% of at least one prolamine and at least one plasticizer, the plasticizer/aqueous-alcoholic prolamine solution ratio being between 0.1/1 and 0.5/1.

4. Biodegradable capsules according to claim 3, **characterized in that** the said polymeric film comprising one prolamine is preferably obtained from a starting solution which comprises (on a weight/weight basis) 40 to 60% aqueous-alcoholic solution, 25 to 30% prolamine and 10 to 30% plasticizer.

5. Biodegradable capsules according to any one of claims 1 to 4, **characterized in that** the plasticizer is a mixture of sorbitol and glycerol in a ratio of between 1/1 and 2.5/1, preferably between 2/1 and 2.5/1.
